(19) Europäisches Patentamt / European Patent Office / Office européen des brevets

(11) **EP 2 739 646 B1**

(12) **EUROPEAN PATENT SPECIFICATION**

(45) Date of publication and mention
of the grant of the patent:
**24.01.2018  Bulletin 2018/04**

(21) Application number: **12721415.3**

(22) Date of filing: **09.05.2012**

(51) Int Cl.:
***C07K 14/62*** *(2006.01)*

(86) International application number:
**PCT/EP2012/001992**

(87) International publication number:
**WO 2012/152439 (15.11.2012 Gazette 2012/46)**

(54) **COMPREHENSIVE BUFFER SYSTEM FOR RENATURING HUMAN PROINSULIN OR PROINSULIN DERIVATIVES**

UMFASSENDES PUFFERSYSTEM ZUR RENATURIERUNG VON MENSCHLICHEM PROINSULIN ODER PROINSULINDERIVATEN

SYSTÈME TAMPON COMPLET POUR LA RENATURATION DE LA PRO-INSULINE HUMAINE OU DE DÉRIVÉS DE PRO-INSULINE HUMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2011  EP 11003823**

(43) Date of publication of application:
**11.06.2014  Bulletin 2014/24**

(73) Proprietor: **Hermann, Stefan**
**40237 Düsseldorf (DE)**

(72) Inventors:
• **ECKERT, Kelvin**
**10245 Berlin (DE)**
• **HERMANN, Stefan**
**40237 Düsseldorf (DE)**
• **NOACK, Janina**
**14478 Potsdam (DE)**
• **SCHWEDE, Matthias**
**14476 Potsdam (DE)**

(74) Representative: **Gille Hrabal**
**Brucknerstrasse 20**
**40593 Düsseldorf (DE)**

(56) References cited:
EP-A1- 2 374 888       EP-A2- 0 668 292
WO-A1-99/33988        WO-A2-2009/133529
US-A- 5 514 646

• OLMOS J ET AL: "Production in Escherichia coli of a rat chimeric proinsulin polypeptide carrying human A and B chains and its preparative chromatography", JOURNAL OF BIOTECHNOLOGY, ELSEVIER SCIENCE PUBLISHERS, AMSTERDAM, NL, vol. 38, no. 1, 30 November 1994 (1994-11-30), pages 89-96, XP023788198, ISSN: 0168-1656, DOI: 10.1016/0168-1656(94)90150-3 [retrieved on 1994-11-30]
• CHANG S-G ET AL: "HUMAN INSULIN PRODUCTION FROM A NOVEL MINI-PROINSULIN WHICH HAS HIGH RECEPTOR-BINDING ACTIVITY", BIOCHEMICAL JOURNAL, THE BIOCHEMICAL SOCIETY, LONDON, GB, vol. 329, 1 January 1998 (1998-01-01), pages 631-635, XP001037629, ISSN: 0264-6021
• VALLEJO LUIS FELIPE ET AL: "Strategies for the recovery of active proteins through refolding of bacterial inclusion body proteins", MICROBIAL CELL FACTORIES, BIOMED CENTRAL, LONDON, NL, vol. 3, no. 1, 2 September 2004 (2004-09-02), page 11, XP021007184, ISSN: 1475-2859, DOI: 10.1186/1475-2859-3-11

**Description**

The present invention relates to an improved method for obtaining a recombinantly expressed precursor of human insulin, human proinsulin, or a precursor of a human insulin derivative having correctly bonded cystine bridges by recombinant expression, encompassing the steps of solubilisation of isolated and purified inclusion bodies, purification of pro-insulin or a proinsulin derivate from solubilised inclusion bodies using ion exchange chromatography, refolding of proinsulin or a proinsulin derivate comprising native disulfide bonds, precipitation of proinsulin or a proinsulin derivate containing non-native disulfide bonds, as well as the steps of concentration and purification of proinsulin or a proinsulin derivate comprising native disulfide bonds using ion exchange chromatography.

Human proinsulin is a protein of about 10 kD, consisting of an A chain, comprising 21 amino acid residues, and a B chain, comprising 30 amino acid residues, both of which are present in the biologically active, mature form of human insulin, and a linker region, the so called C peptide, which comprises 35 amino acid residues and which is absent from the mature human insulin. Six cysteine residues are present within the A and B chains at positions A6, A7, A11 and A20 as well as B7 and B19, whereby the letters represent the respective A and B chains and the numbers represent the position of the amino acid residue, counting from the amino to the carboxyl end of the respective amino acid chain. In the mature, biologically active human insulin, the A and B chains are linked by disulfide bridges between residues A7 and B7 and between residues A 20 and B 19. An intrachain disulfide bond is formed between residues A6 and A11. In the following, the term "correctly bonded cystine bridges" refers to these disulfide bonds, present in the biologically active human insulin.

Known processes for the preparation of human insulin are based on the recombinant expression of a precursor of human insulin (proinsulin) or a human proinsulin derivative in suitable microorganisms (e.g. EP 0 055 945). The formation of correct disulfide bonds in human proinsulin or proinsulin derivates generated by recombinant expression depends, however, on the microorganism used for expressing the protein. In particular, proinsulins prepared from genetically modified *Escherichia coli* (*E. coli*) cells do not exhibit correctly bonded cystine bridges. The use of *E. coli,* however, for the production of heterologous proteins, such as insulin, has been widespread for the past decades (Pines and Inouye, 1999, Mol. Biotechnol., 12, 25-34), due to a favourable cost-value relation. *E. coli* bacteria are easy to cultivate, rapidly generate biomass and are accessible to high density fermentation, reflecting the fact that, compared to eukaryotic expression in systems such as yeast cells, bacterial expression harbours the advantages of good scalability and far shorter fermentation runs. These advantages are counterbalanced by the fact that *E. coli* cells are unable to perform most of the posttranslational modifications of proteins, e.g. the formation of correct cystine bridges (see above), required for the functioning of most eukaryotic proteins. Moreover, high-yield expression in *E. coli* often results in protein aggregation in the form of inclusion bodies, which are intracellular aggregates of insoluble, biologically inactive proteins. While the target protein is present within these inclusion bodies in a relatively concentrated form, it is necessary to separate other inclusion body proteins from the protein of interest, which, after this initial purification, has to be renatured and converted to its biologically active form. Generally, this *in vitro* renaturation process involves a complex series of biochemical reactions, the nature and order of which is highly dependent on the structural requirements of the protein to be converted (Rudolph and Lilie, 1986, FASEB, 10: 49-56; DeBernadez-Clark, 2001, Curr. Opinion Biotech, 12: 202-207). Accordingly, starting from a fully denatured precursor isolated from inclusion bodies, the final yields of the biologically active, "native" form of a renatured protein often are very low. Hence, even a modest increase in the yield of biologically active, renatured protein is considered a significant improvement of procedure, often associated with a clear commercial advantage. Common problems encountered in the production process of recombinant proteins include misfolding of proteins, protein aggregation as well as substantial protein loss associated with a multi-step process. While the problems of misfolding and aggregation are tractable by way of providing optimal refolding conditions, possibly including folding promoters such as high or low molecular weight chaperones, and low protein concentrations, protein loss can be prevented by optimizing reactions such as reducing processing steps to a minimum, thereby preventing e.g. adsorption to solid phase materials during multiple purification steps.

Thus, in the case of recombinantly produced human insulin or a derivative thereof, starting from human proinsulin or human proinsulin derivative expressed in a prokaryotic system, e.g. *E. coli,* a considerably large number of process steps have to be performed for obtaining a biologically active human insulin or human insulin derivative. A method for producing human insulin from *E. coli* cells (EP 0 055 945) is based on the following process steps: fermentation - cell lysis - purification of the inclusion bodies - isolation of the fusion protein - cyanogen halide cleavage of the fusion protein - isolation of the proinsulin cleavage product - S-sulfonation of cysteine residues (sulfitolysis) - chromatographic purification of the sulfonated proinsulin- refolding of proinsulin for the formation of correctly bonded cystine bridges - chromatographic purification of the proinsulin comprising correctly bonded cystine bridges - concentration - chromatographic purification of concentrated proinsulin - enzymatic cleavage of proinsulin to generate mature human insulin - chromatographic purification. The larger the number of process steps required, the more significant is the loss of protein, which eventually leads to a low yield of insulin. In EP 0 055 945, a loss of up to 40% of the proinsulin product starting from the stage of the isolated fusion protein to the stage of purification of the proinsulin product is described, with further substantial losses occurring during subsequent processing steps before obtaining the final product.

Among the reasons for the loss of proinsulin are the multiple purification and desalting steps necessitated by the change of buffer systems during the protein production process. Different pH conditions are required for solubilisation of inclusion bodies (alkaline pH), reduction of pre-existing disulfide bonds (alkaline pH), shuffling (alkaline pH, narrow range), precipitation of wrongly folded intermediates (neutral pH), purification and concentration (acidic pH). Therefore, the use of different buffering compounds has been described in the patents (e.g. EP0489780, EP0347781, CA02315750) and in the scientific literature (e.g. Winter et al., 2002; Son et al., 2008). Patents EP0489780 and EP0347781 describe the use of a tris(hydroxymethyl) aminomethane (i.e. Tris)- buffered solution for solubilisation of inclusion bodies and sulfonation of the solubilised proteins, followed by anion exchange chromatography in either glycine buffer (EP0489780) or Tris buffer (EP0347781), followed by refolding in glycine buffer (EP0489780; EP0347781) and subsequent chromatographic purification by ion exchange in glycine buffer (EP0347781). CA02315750 discloses a Tris buffered solution (20 mM) for the steps of solubilisation of inclusion bodies and protein sulfonation, while the refolding step is conducted in a buffer containing 50 mM glycine. Winter et al. (Analytical Biochemistry, 2002, 310, 148-155) disclose the denaturation of human proinsulin in a Tris buffered system, followed by refolding in an equimolar Tris/glycine buffered solution. Son et al. (J. Microbiol. Biotechnol., 2008, 18(5); 983-989) describe the solubilisation of inclusion bodies in a Tris/glycine buffered solution and refolding in 10 mM glycine buffer. Thus, in those protocols, the exchange of buffer solutions between the process steps is required.

Furthermore, in protein purification procedures starting from inclusion bodies, solubilisation and refolding steps are usually carried out in the presence of a chaotropic auxiliary. A chaotrope is a substance which disrupts the three-dimensional structure of proteins by interfering with non-covalent forces in proteins, such as hydrogen bonds and van der Waals interactions, occurring between amino acids within a chain and between chains of a protein. Examples of chaotropic substances are ammoniumsulfate, ethylenecarbonate, thiocyanate, dimethylsulfonate, thiourea, lithiumperchlorate, guanidinium hydrochloride and urea. Predominantly, two of these chaotropic auxiliaries are used for the denaturation of proteins: guanidinium hydrochloride (GdnHCl) and urea. A disadvantage of using urea is the generation of ammonium cyanate, which is in equilibrium with urea in highly concentrated solutions (such as 6-8 M) of the chaotrope in an aqueous buffer, and which is capable of modifying proteins via carbamylation. During carbamylation of proteins, primary amines are modified irreversibly (N termini and e-amino groups of lysines; see: Cole, 1961, J. Biol.Chem. 236, 2670-2671), thus possibly resulting in unwanted modifications of protein structure (Oimomi et al., 1987, Nephron, 46, 63-66). Moreover, modifications of hydroxyl, sulfhydryl or imidazole moieties also are possible, but at alkaline pH, this reaction is reversible. Carbamylation can be prevented by adding cyanate scavengers to the solution, such as ethylene diamines, which work in the range of neutral pH and slightly below (US 4,605,513) or non-ethylene diamine compounds, such as glycinamide, histidine, 4-hydroxyl proline and various dipeptides (US 7,459,425). Albeit the efficiency of carbamylation prevention of the added compounds is excellent and ranges from 80% to 100% protection depending on the assay used, the compounds have to be added to the aqueous medium and cleared from it in the subsequent purification steps.

EP 2 374 888 A1 relates to methods of producing human recombinant insulin by recombinant expression in E.coli. Examples 8 and 9 relate to the purification of inclusion bodies wherein the inclusion bodies are dissolved in 8M urea containing 50mM glycine. Afterwards the solution was clarified by high speed ultracentrifugation and reduced insulin was refolded in a buffer comprising 10mM glycine (see e.g. Example 10). The concentration of the refolded insulin is routinely carried out by ultracentrifugation or using absorption chromatography.

US 5 514 646 A discloses the production of insulin analogues by recombinant DNA techniques. Example 28 describes the solubilisation of inclusion bodies in the presence of cysteine and urea, purification by ion exchange chromatography, and the refolding at pH 10.6 in the presence of cysteine. The refolding is quenched by adjusting the pH to 2.4, improperly folded proinsulin is separated by hydrophobic interaction chromatography and insulin is further purified by cation exchange chromatography followed by HPLC.

EP 0 668 292 A2 describes the isolation of insulin that is correctly post-translationally processed by reacting pro-insulin with a mercaptan in the presence of a chaotropic agent and purification after absorption to hydrophobic resin.

WO 2009/133529 A2 relates to the refolding of proinsulin wherein the forming insulin precursor/analog having correctly bonded cystine bridge involves mixing insulin precursor having incorrectly bonded cystine bridge with buffer such as a glycine buffer containing cysteine/its hydrochloride and chaotropic auxiliary and adding diluent.

Surprisingly, it has now been found that the yield of correctly folded proinsulins or proinsulin derivatives can be increased and the overall time for the production process can be reduced significantly by conducting all steps as described herein in the same aqueous buffer system comprising an organic amphoteric substance containing at least two functional groups, comprising at least one amino group and one carboxylic acid group, and wherein the functional groups exhibit pKa values which lie between 2.0 and 10.0 and wherein the difference between the pKa values of at least one amino group and at least one carboxylic acid group of the amphoteric substance is at least 7. This organic amphoteric substance is, in addition to its function as an advantageous buffering compound, capable of reducing harmful by-products associated with the presence of high concentrations of urea.

**Detailed description of the invention**

The present invention is a method for producing proinsulin or a proinsulin derivate by recombinant expression in a prokaryotic organism comprising the steps

A) Solubilisation of inclusion bodies,
B) Purification of proinsulin or a proinsulin derivate from solubilised inclusion bodies using ion exchange chromatography,
C) Refolding proinsulin or a proinsulin derivate comprising native disulfide bonds,
D) Precipitation of proinsulin or a proinsulin derivate containing non-native disulfide bonds,
E) Concentration and purification of proinsulin or a proinsulin derivate comprising native disulfide bonds using ion exchange chromatography,

wherein all steps are carried out in an aqueous buffer comprising an organic amphoteric substance at a concentration between 0.001 M and 0.5 M containing at least two functional groups, comprising at least one amino group and one carboxylic acid group, and wherein the functional groups exhibit pKa values which lie between 2.0 and 10.0 and wherein the difference between the pKa values of at least one amino group and at least one carboxylic acid group of the amphoteric substance is at least 7.

An amphoteric substance is one that can react as either an acid or a base. Preferably, the amphoteric substance of the present invention is an organic substance which contains at least two functional groups, comprising at least one amino group and one carboxylic acid group. The functional groups exhibit pKa values which lie between 2.0 and 10.0 and the difference between the pKa values of at least one amino group and at least one carboxylic acid group of the amphoteric substance is at least 7. A pKa spread of at least 7 was found to be essential for achieving high yield with good purity of a proinsulin or a proinsulin derivate comprising native disulfide bonds,

The term pKa is a constant referring to the acid-ionization constant, $K_a$, to which is related by the equation:

$$pKa = -\log_{10} Ka \ (1).$$

Ka is the equilibrium constant for a dissociation reaction in the context of acid base reactions, hence it is a measure of the strength of an acid in solution. For the present invention, the term "pKa value" refers to a value obtained under conditions relevant for standard biochemical procedures. It is well known that the highest buffering capacity b is observed at a constant buffer concentration when

$$pH = pKa \ (2).$$

The concentration of the organic amphoteric substance in the aqueous medium is between 0.001 M and 0.5 M, preferably between 0.01 M and 0.1 M, most preferred is a concentration between 0.02 M and 0.05 M to provide for sufficiently high buffering capacity, b, to which the concentration ($C_{buf}$) and the pKa (as $K_a$) value of the organic amphoteric substance are related by the following equation:

$$\beta = 2.303 \left( \frac{K_w}{[H^+]} + [H^+] + \frac{C_{buf} K_a [H^+]}{(K_a + [H^+])^2} \right) \ (3)$$

$K_w$ refers to the dissociation constant of pure water; at 25 °C $K_w$ is approximately equal to $1.0 \times 10^{-14}$. Preferred are amphoteric substances with the pKa value of one carboxylic acid group ranging from 2.0 to 2.5 and the pKa value of amino group ranging from 9.5 to 10.5.

By exhibiting at least two pKa values falling within the indicated ranges, excellent buffer capacity is achieved in the pH ranges required for the purification of proinsulin and derivatives thereof from inclusion bodies. Specifically, the process steps of (A) solubilisation of inclusion bodies and (B) purification of proinsulin or proinsulin derivate from solubilised inclusion bodies using ion exchange chromatography as well as (C) refolding proinsulin or a proinsulin derivate comprising native disulfide bonds are conducted within the alkaline pH range. In particular, step A is carried out at a pH ranging from pH = 9.0 to pH = 11.5, especially preferred at pH = 9.5. The result of inclusion body solubilisation at high alkaline pH is soluble denatured protein without significant amounts of secondary structure. Step (B) is a chromatographic purification step required for separation of the fully denatured and reduced precursor protein from other inclusion body proteins. Bead- or membrane-based anion exchange chromatography, preferably employing quaternary amines as functional groups, is conducted at pH = 9.0 to pH = 11.5, especially preferred at pH = 9.5. In this step, proinsulin or proinsulin derivative are purified to some extent from the chaotropic denaturant as well as the reducing agent, which do not bind to the functional groups of the anion exchanger at the pH applied. The chaotropic denaturant as well as reducing agent have to be added back to keep the proinsulin or proinsulin derivative in a fully reduced and denatured state until initiation of the refolding reaction. Small amounts of both compounds are required in this subsequent reaction (step C). For the refolding reaction (C), a pH range of pH = 9.5 to pH = 10.5 is preferred, especially preferred at pH = 10.5. The reaction is stopped (D) by adjusting the pH to an acidic value, which is preferably between pH = 4.8 and pH = 5.6, most preferred to pH = 5.2, whereby proinsulin or proinsulin derivate containing non-native disulfide bonds are precipitated and can be isolated by a simple centrifugation or filtration step. In the subsequent step (E), bead- or membrane-based ion exchange chromatography is used to purify proinsulin or proinsulin derivate containing native disulfide bonds utilizing cation exchange at a pH ranging from pH = 2.5 to pH = 4.5, especially preferred at pH = 3.5, followed by further purification (F) using anion exchange at a pH ranging from pH = 8.5 to pH = 10.5, especially preferred at pH = 9.5.

The preferred organic amphoteric substances of the invention to be used as a buffer system are amino acids. Preferably, the organic amphoteric substance exhibits a suitable pKa spread and an excellent solubility in a number of different solvents, in particular water, at a range of different temperatures, to yield a buffer compound with a sufficient buffer capacity b in the required pH ranges to be used in the context of the protein processing and purification procedure of the present invention. In particular, it is an amino acid exhibiting a water solubility of greater than 150 g/l at 20°C, which is required to achieve good buffer capacity.

Specifically suitable are the amino acids glycine, arginine, alanine, beta-alanine, serine or proline. In the present invention, it has been found that the amino acids glycine, alanine and serine are the most adequate organic amphoteric buffer substances in terms of buffering capacity, pKa spread and water solubility, most preferred is glycine. Especially, regarding buffering capacity, it was observed that glycine is a high strength compound in comparison to other amino acid buffers. Thus, concentration and pH-dependent tendency for aggregation, which proinsulin is prone to at neutral to slightly acidic pH even at low concentrations of protein, is diminished significantly using glycine as a buffering compound within the specified concentration range.

The most preferred organic amphoteric substance, glycine, exhibits high water solubility (25g/100g water at 25°C) and the most favourable pKa spread (2.35; 9.78). By comparison, other amino acids with high water solubility, such as arginine (15g/100g water at 25°C), alanine (16.5 g/100 g water at 25°C), serine (5 g/100 g water at 25°C), proline (162 g/100 g water at 25°C) and cysteine (>50 g/100 g water at 25°C) also can be used advantageously as amphoteric substances. Glycine, however, is the most preferred amino acid buffer substance in comparison, for the following reasons: for arginine and proline, the spread of their respective pKa values is not as suitable (arginine: 2.01, 9.4, 12.48; proline: 2.0, 10.6), for cysteine, the pKa spread (2.05, 10.25, 8.00) is more satisfactory, but it comprises a side chain with a functional group, which could react with the protein to be purified under the conditions utilized. Furthermore, considering also the ease and cost efficiency of preparation, glycine is considered inexpensive (unlike serine, alanine and beta-alanine).

In addition, the organic amphoteric substance of the invention efficiently acts as a cyanate scavenger, maintaining the cyanate concentration in an aqueous solution containing urea in a concentration range of 5 M to 8 M at concentrations lower that 1.5 mM for a time period of at least 7 days. This is particularly advantageous, as primary amines (e.g. present at the N termini and the side chains of lysine and arginine) are prone to irreversible modification by carbamylation at the pH range required for solubilisation of inclusion bodies and refolding (i.e. high alkaline pH values). Carbamylation of these residues, resulting in N-terminal carbamine, epsilon-carbamyllysine and N-carbamylarginine, disrupts their capability to form native hydrogen bonds, which stabilize intra- and interchain interactions, thus resulting in modifications of protein structure, function and/or stability. While polyamines, especially ethylene diamines, have been described as efficient cyanate scavengers (US 4,605,513; US 76 459, 425 B2) here it is surprisingly found that the amino acid glycine can scavenge the decomposition products associated with high urea concentrations (i.e. ammoniumcyanate) with a comparable efficiency, thereby leading to an improved situation during the solubilisation and refolding step in comparison with other patented procedures, wherein an additional molecular scavenger has to be added and subsequently removed in the overall production process for e.g. proinsulin or a proinsulin derivate comprising native disulfide bonds.

The presence of an efficient cyanate scavenger in the buffer thus enables the use of urea as a chaotropic auxiliary for solubilisation of inclusion bodies containing human proinsulin at high alkaline pH values. As an alternative, among others, guanidinium hydrochloride could be utilized. Urea, however, is the most preferred chaotropic auxiliary, as it is an uncharged molecule (in contrast to guanidinium hydrochloride) and therefore compatible with chromatographic purification steps based on ion exchange, which are employed in the present invention in steps (B), (E) and (F). These chromatographic techniques bear relatively high economic (cost of material, flow volumes) and procedural (time) advantages compared to other concentration- and purification-techniques such as dia- and/or ultrafiltration.

While the amino acids glycine, alanine and serine are the most suitable organic amphoteric buffer substances in terms of buffering capacity, pKa spread and water solubility, with glycine being the most preferred compound, for the precipitation step (D), buffering capacity in the mildly acidic to neutral range is required. In the present invention, it was found surprisingly, that by adding small concentrations of a second buffer substance which has at least one pKa value within the central 50% of the range defined by the pKa values of at least one amino group and at least one carboxylic acid group of the amphoteric substance, the pH could be stabilized efficiently to allow for quantitative precipitation of misfolded proteins while not affecting significantly proteins with correctly bonded cysteine bridges. The preferred pH range for (D) is between pH = 4.8 and pH = 5.6. Therefore, substances characterized by at least one pKa between 4.5 and 5.9 are best suited for buffering in this range. In particular, it was found that mono- and dicarboxylic acids of the general chemical formulae R-COOH and HOOC-R-COOH, respectively, including e.g. acetic, propanoic, butyric, valeric or benzoic acid and e.g. succinic, glutaric, adipic, pimelic, suberic, or azelaic acid, can efficiently stabilize the pH within the range most suitable for precipitation of misfolded proinsulin or derivatives thereof. Most preferred, a dicarboxylic acid is added to the aqueous medium comprising one first buffer substance at the end of the refolding reaction (step D). Dicarboxylic acids are preferred over monocarboxylic acids as they exhibit two pKa values which allow for a greater spread of buffering capacity in the indicated pH range and hence provide for more flexibility as to the pH at which precipitation is carried out. Moreover, water solubility of dicarboxylic acids in general is greater for comparable C- chain lengths, e.g. glutaric acid (HOOC-$(CH_2)_3$-COOH; 64.0 g/100ml) is more soluble than valeric acid ($C_4H_9$COOH; 4.97g/100ml). Especially preferred in the context of the invention are the dicarboxylic acids succinic acid and glutaric acid, with the highest preference given to succinic acid due to its most favourable spread of pKa values ($pK_{a1}$ = 4.2; $pK_{a2}$ = 5.6).

Thus, by using a comprehensive buffer system for renaturing human proinsulin or proinsulin derivatives, wherein the buffer provides excellent buffering capacity at the divergent pH ranges necessary for adequate processing of the protein in question, while at the same time acting as a scavenger for decomposition products of essential buffer components, a simple and shortened procedure can be used for the renaturation of human proinsulin with increased yield of the native biologically active form.

In the following, the present invention will be described with respect to working examples. Reference is made in this respect to the following figures:

FIG. 1: Optimization of the precipitation conditions: Semi-quantitative gel densitometry revealed that at pH=5.2 most of the misfolded proinsulin, but only minimal amounts of correctly folded proinsulin, was precipitated.

Fig. 2: Concentration and purification of the correctly folded proinsulin: Analytical RP-HPLC of purified human proinsulin after the final purification step.

EXAMPLES

1. Scavenger activity of 50 mM glycine buffer, pH 9.5, calibration curve

Cyanate was added to a buffer solution containing 200 mM sodiumdiarsinate at a pH of 9, 9.5 or 11. Utilizing a modification of the method described by Warner (1942), copper-pyridine-cyanate complexes could be detected by measuring photometrically the absorbance at a wavelength lambda = 700 nm in a BioRad Smartspec Plus spectrophotometer. By using cyanate concentrations within a range relevant in the context of protein purification (i.e.: 0, 1.2, 2.5, 3.7, 4.9, 7.4 mM) a near linear relationship was obtained with a coefficient of determination, $R^2$, approximating 0.97.

The scavenging activity of 50 mM glycine buffer, pH 9.5, was determined in the presence of 5 M urea during the time course of 7 d at room temperature. For controls, no glycine was added.

|  | Cyanat [mM] |
| --- | --- |
| pH 9 | 0 |
| pH 11 | 0 |
| pH 9, 7 d | 9 |
| pH 11, 7 d | 3 |
| Glycin, pH 9,5, 0 d | 0 |
| Glycin, pH 9,5, 2 d | 0 |
| Glycin, pH 9,5, 3 d | 0 |

(continued)

| | Cyanat [mM] |
|---|---|
| Glycin, pH 9,5, 4 d | 1 |

From this data a calibration curve can be generated by using the modified method according to Warner (J. Biol. Chem., 142, 705; 1942).

## 2. Solubilisation of inclusion bodies

Pre-purified inclusion bodies isolated from approximately $1.5*10^{13}$ *E. coli* cells expressing the appropriate construct (e.g. human proinsulin or a derivative thereof) were dissolved in 30 ml of a 50 mM glycine buffer at pH 9.5 and solubilised by adding solid urea to a final concentration of 7.5 M. Moreover, 100 mM beta-mercaptoethanol was added to the solution to provide a strongly reducing environment. Incubation was conducted for 1 hour at 20-37°C using slight agitation (stirring or shaking); subsequently, insoluble cellular debris was discarded using centrifugation or, as an alternative, microfiltration.

## 3. Purification of fully denatured and reduced proinsulin

The supernatant/filtrate was purified directly by column-based anion exchange chromatography, using membrane based exchangers exhibiting a high dynamic binding capacity (e.g. Mustang-Q, binding capacity: 34 mg/ml; 5 ml solidphase material). During the washing of the loaded column using 50 mM glycine buffer at pH 9.5, the concentrations of urea and beta-mercaptoethanol were lowered to 5M urea and 5 mM beta-mercaptoethanol. Fully denatured and reduced proinsulin was eluted using a linear gradient of NaCl (0-1000 mM, in 50 mM glycine buffer at pH 9.5 supplemented with 5 M urea and 5 mM [beta]-mercaptoethanol). Concentration of the precursor in the combined elution fractions was determined either by using an assay according to Bradford (Bradford, 1976) or by spectrophotometry (absorbance assay at 1 =280nm; fully reduced denatured proinsulin has a molar extinction coefficient of [epsilon]=5960 l/(mol*cm)) and varied between 5 to 6 g/l.

## 4. Renaturation of the purified proinsulin: refolding reaction and precipitation of misfolded proinsulins

The elution fractions were diluted approximately 20-fold to a protein concentration of 0.1 - 1,5 g/l in refolding buffer, which contained 50 mM glycine, pH 10.5, and urea adjusted to a final concentration of 1 M. The reducing agent carried over from anion exchange chromatography (approximately 0.25 mM beta-mercaptoethanol) was complemented by adding 1 mM cystine (oxidized cysteine) for formation of a mixed redox system. The refolding mixture was incubated for 12-14 hours either at room temperature or at 4°C using slight agitation (stirring) under an air atmosphere. After the indicated time, succinic acid was added to the reaction mixture to a final concentration of 5 mM for pH stabilization prior to stopping the refolding reaction by adjusting the pH to 5.2 using 1 M HCl. Precipitation of misfolded proinsulin molecules was allowed to proceed for 1 hour and RT under continuous stirring. Precipitates were separated by paper filtration using Whatman No. 1 filter paper. Precipitate can be denatured again in 50 mM glycine buffer, pH 9.5 containing urea at 5 M and beta-mercaptoethanol at 100 mM, upon which the fully denatured and reduced proinsulin can be subjected to an additional folding reaction to enhance overall yield.

## 5. Optimization of the precipitation conditions.

DE3501641 disclosed the precipitation of misfolded proinsulins by acidifying to conditions slightly below their isoelectric point. However, the exact conditions vary with the particular amino acid sequence and have to be determined empirically.

Proinsulin folded according to example 4 was supplemented with 5 mM succinic acid. Aliquots were acidified with hydrochloric acid to pH=5.0, pH=5.2, pH=5.4 and pH=5.6, respectively, and the mixture allowed to incubate for another hour under gentle stirring. The precipitates formed were removed by centrifugation (10 min at 15000 g) and precipitates (P) and supernatants (S) were analyzed by SDS-PAGE.

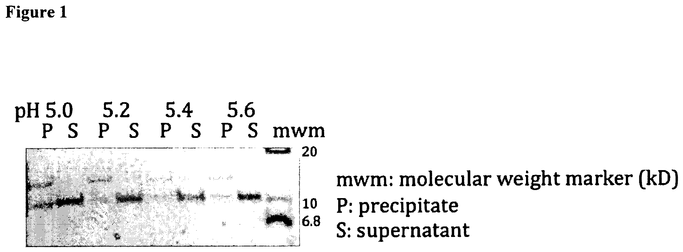

According to Fig. 1, semi-quantitative gel densitometry revealed that at pH=5.2 most of the misfolded proinsulin, but only minimal amounts of correctly folded proinsulin, was precipitated.

6. Concentration and purification of the correctly folded proinsulin

The pH of the filtrate obtained from the filtration step was adjusted to 3.5 using 1 M HCl and loaded directly onto a pre-equilibrated cation exchange column, e.g. S-Sepharose (GE Healthcare) with a bed volume of 10 ml. At this pH, proinsulin is positively charged and can be eluted by reversing the charges via a rapid pH change. Accordingly, the cation exchange column was washed using 20mM glycine buffer, pH 3.5, (i.e. low strength), followed by elution in 100 ml of 50mM glycine buffer, pH 9.5 (high strength). The first 25 ml were discarded, as they contained no proinsulin. The combined proinsulin-containing fractions had a final concentration of 6-8 g/l. The elution fractions were subjected directly to anion exchange chromatography (e.g. Mustang Q or Q Sepharose) for a final purification step. The column-bound proinsulin was washed with 20 mM Glycin pH=8.5 and eluted with 150 mM NaCl/20 mM Glycine pH=8.5.

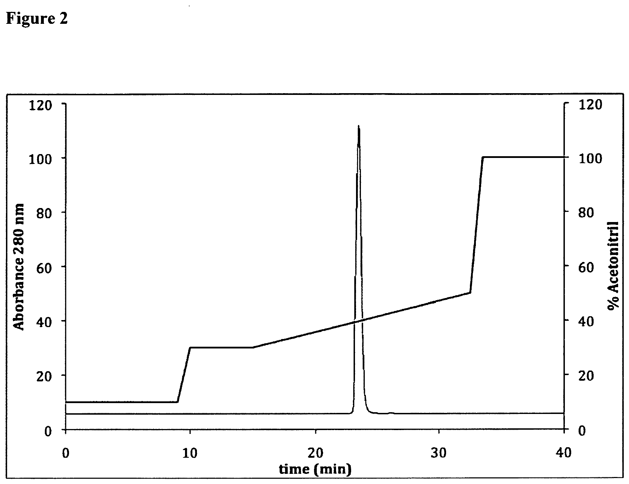

At this point, the proinsulin was judged to be 99 % pure by analytical reverse phase high performance liquid chromatography (RP-HPLC, Fuji Silysia Chromatorex C18, pore size 10 nm, particle size 10 [micro]m, 4,6*250 mm); see figure 2.

The eluted material can be directly subjected to enzymatic processing with trypsin and carboxypeptidase B.

The invention relates to an improved processing and purification method for the production of proinsulin or derivatives thereof starting from the solubilisation of inclusion bodies isolated from a prokaryotic expression system, such as E. coli, whereby all processing (i.e. refolding, precipitation, concentration) and purification steps are carried out in the same aqueous buffer system comprising an organic amphoteric substance containing at least two functional groups, which contain at least an amino and a carboxylic acid group, and which is characterized by a difference in the pKa values of its at least one amino group and at least one carboxylic acid group of at least 7. Furthermore, the organic amphoteric substance is an efficient scavenger of cyanate, thereby preventing irreversible carbamylation of primary amines present in proinsulin or the derivates thereof, which has been observed in the presence of high concentrations of urea. Thus, the disadvantage associated with this widespread chaotropic auxiliary is obliterated, allowing the utilization of efficient and uncomplicated chromatography techniques for purification steps subsequent to solubilisation and refolding. The use of an efficient buffer substance in all processing steps from inclusion body solubilisation up to the re-folded, concentrated and purified precursor of insulin or a derivate thereof, which at the same time acts as an agent preventing protein modification by carbamylation, thereby abolishing the need to add an additional compound with scavenging activity, leads to significant advantages regarding the efficiency and efficacy of the procedure compared to previously employed production methods.

## Claims

1. A method for producing proinsulin or a proinsulin derivate by recombinant expression in a prokaryotic organism comprising the steps

    A) Solubilisation of inclusion bodies,
    B) Purification of proinsulin or a proinsulin derivate from solubilised inclusion bodies using ion exchange chromatography,
    C) Refolding proinsulin or a proinsulin derivate comprising native disulfide bonds,
    D) Precipitation of proinsulin or a proinsulin derivate containing non-native disulfide bonds,
    E) Concentration of proinsulin or a proinsulin derivate comprising native disulfide bonds using ion exchange chromatography,
    F) Purification of proinsulin or a proinsulin derivate comprising native disulfide bonds using ion exchange chromatography,

    wherein all steps are carried out in an aqueous buffer comprising an organic amphoteric substance at a concentration between 0.001 M and 0.5 M containing at least two functional groups, comprising at least one amino group and one carboxylic acid group,
    wherein the functional groups exhibit pKa values which lie between 2.0 and 10.0 and wherein the difference between the pKa values of at least one amino group and at least one carboxylic acid group of the amphoteric substance is at least 7.

2. A method according to claim 1, wherein steps A) and B) are carried out at a pH between pH = 9.0 and pH = 11.5.

3. A method according to any of the claims 1 or 2, wherein step C) is carried out at a pH between pH = 9.5 and pH = 10.5.

4. A method according to any of the claims 1 to 3, wherein step D) is carried out at a pH between pH = 4.8 and pH = 5.6.

**5.** A method according to any of the claims 1 to 4, wherein step E) is carried out at a pH between pH = 3.5 and pH = 4.5.

**6.** A method according to any of the claims 1 to 5, wherein step F) is carried out at a pH between pH = 8.5 and pH = 10.5.

**7.** A method according to claim 1 or 6, wherein the organic amphoteric substance is an amino acid with a water solubility of greater than 150 g/l at 20°C.

**8.** A method according to claim 1 or 7, wherein the organic amphoteric substance is one or more of: glycine, arginine, alanine, beta-alanine, serine, cysteine or proline.

**9.** A method according to claim 1 or 8, wherein the organic amphoteric substance maintains the cyanate concentration in an aqueous solution containing urea in a concentration range of 5 M to 8 M at concentrations lower that 1.5 mM.

**10.** A method according to any of the claims 1 to 9, wherein in step D) a second buffer substance is added to the aqueous buffer, the second buffer substance has at least one pKa value within the central 50% of the range defined by the pKa values of at least one amino group and at least one carboxylic acid group of the amphoteric substance.

**11.** A method according to claim 10, wherein at least one pKa value of the second buffer substance is between 4.5 and 5.9.

**12.** A method according to claim 10 or 11, wherein the second buffer substance is a monocarboxylic acid, in particular, acetic, propanoic, butyric, valeric or benzoic acid.

**13.** A method according to claim 10 to 12, wherein the second buffer substance is a dicarboxylic acid, in particular, succinic, glutaric, adipic, pimelic, suberic, or azelaic acid.

**14.** A method according to claim 10 to 12, wherein the second buffer substance is succinic acid.

**Patentansprüche**

**1.** Ein Verfahren zur Herstellung von Proinsulin oder eines Proinsulinderivates durch rekombinante Expression in einem prokaryotischen Organismus, das die Schritte

A) Solubilisierung von Einschlusskörpern,
B) Reinigung von Proinsulin oder eines Proinsulinderivats aus solubilisierten Einschlusskörpern unter Verwendung von Ionen-Austausch-Chromatographie,
C) Rückfaltung von Proinsulin oder eines Proinsulinderivats, die native Disulfidbindungen umfassen,
D) Ausfällung von Proinsulin oder eines Proinsulinderivats, die nichtnative Disulfidbindungen enthalten
E) Konzentration von Proinsulin oder eines Proinsulinderivats, die native Disulfidbindungen umfassen, unter Verwendung von Ionen-Austausch-Chromatographie
F) Reinigung von Proinsulin oder eines Proinsulinderivats, die native Disulfidbindungen umfassen, unter Verwendung von Ionen-Austausch-Chromatographie,

umfasst,
worin alle Schritte in einem wässrigen Puffer durchgeführt werden, der eine organische amphotere Substanz in einer Konzentration zwischen 0,001 M und 0,5 M umfasst, die mindestens zwei funktionelle Gruppen enthält, die mindestens eine Aminogruppe und eine Carbonsäuregruppe umfassen,
worin die funktionellen Gruppen pKa-Werte aufweisen, die zwischen 2,0 und 10,0 liegen und worin die Differenz zwischen den pKa-Werten mindestens einer Aminogruppe und mindestens einer Carbonsäuregruppe der amphoteren Substanz mindestens 7 beträgt.

**2.** Verfahren gemäß Anspruch 1, worin die Schritte A) und B) bei einem pH-Wert zwischen pH = 9,0 und pH = 11,5 durchgeführt werden.

**3.** Verfahren gemäß einem der Ansprüche 1 oder 2, worin der Schritt C) bei einem pH-Wert zwischen pH = 9,5 und pH = 10,5 durchgeführt wird.

**4.** Verfahren gemäß einem der Ansprüche 1 bis 3, worin der Schritt D) bei einem pH-Wert zwischen pH = 4,8 und pH = 5,6 durchgeführt wird.

5. Verfahren gemäß einem der Ansprüche 1 bis 4, worin der Schritt E) bei einem pH-Wert zwischen pH = 3,5 und pH = 4,5 durchgeführt wird.

6. Verfahren gemäß einem der Ansprüche 1 bis 5, worin der Schritt F) bei einem pH-Wert zwischen pH = 8,5 und pH = 10,5 durchgeführt wird.

7. Verfahren gemäß Anspruch 1 oder 6, worin die organische amphotere Substanz eine Aminosäure mit einer Wasserlöslichkeit von mehr als 150 g/l bei 20 °C ist.

8. Verfahren gemäß Anspruch 1 oder 7, worin die organische amphotere Substanz eine oder mehrere ist aus: Glycin, Arginin, Alanin, beta-Alanin, Serin, Cystein oder Prolin.

9. Verfahren gemäß Anspruch 1 oder 8, worin die organische amphotere Substanz in einer wässrigen Lösung, die Harnstoff in einem Konzentrationsbereich von 5 M bis 8 M enthält, die Cyanatkonzentration bei Konzentrationen unterhalb von 1,5 mM aufrecht erhält.

10. Verfahren gemäß einem der Ansprüche 1 bis 9, worin in Schritt D) dem wässrigen Puffer eine zweite Puffersubstanz hinzugefügt wird, und diese zweite Puffersubstanz über mindestens einen pKa-Wert innerhalb der mittleren 50% des durch die pKa-Werte der mindestens einen Aminogruppe und der mindestens einen Carbonsäuregruppe der amphoteren Substanz definierten Bereiches verfügt.

11. Verfahren gemäß Anspruch 10, worin mindestens ein pKa-Wert der zweiten Puffersubstanz zwischen 4,5 und 5,9 liegt.

12. Verfahren gemäß Anspruch 10 oder 11, worin die zweite Puffersubstanz eine Monocarbonsäure ist, insbesondere Essig-, Propion-, Butter-, Valerian-oder Benzoesäure.

13. Verfahren gemäß Anspruch 10 bis 12, worin die zweite Puffersubstanz eine Dicarbonsäure ist, insbesondere Bernstein-, Glutar-, Adipin- Pimelin, Suberin- oder Azelainsäure.

14. Verfahren gemäß Anspruch 10 bis 12, worin die zweite Puffersubstanz Bernsteinsäuresäure ist.

**Revendications**

1. Procédé de production d'une pro-insuline ou d'un dérivé de pro-insuline par expression recombinante dans un organisme procaryote, comprenant les étapes de :

A) solubilisation de corps d'inclusion,
B) purification d'une pro-insuline ou d'un dérivé de pro-insuline des corps d'inclusion solubilisés en utilisant la chromatographie par échange d'ions,
C) replier une pro-insuline ou un dérivé de pro-insuline comprenant des liaisons de disulfures natives,
D) précipitation d'une pro-insuline ou d'un dérivé de pro-insuline contenant des liaisons de disulfures non-natives,
E) concentration d'une pro-insuline ou d'un dérivé de pro-insuline comprenant des liaisons de disulfures natives en utilisant la chromatographie par échange d'ions,
F) purification d'une pro-insuline ou d'un dérivé de pro-insuline comprenant des liaisons de disulfures natives en utilisant la chromatographie par échange d'ions,

dans le lequel toutes les étapes sont effectuées dans un tampon aqueux comprenant une substance amphotère organique à une concentration comprise entre 0,001 M et 0,5 M contenant au moins deux groupes fonctionnels, comprenant au moins un groupe amino et un groupe d'acide carboxylique,
dans lequel les groupes fonctionnels montrent des valeurs de pKa comprises entre 2,0 et 10,0 et dans lequel la différence entre des valeurs de pKa d'au moins un groupe amino et d'au moins un groupe d'acide carboxylique de la substance amphotère est au moins 7.

2. Procédé selon la revendication 1, dans lequel les étapes A) et B) sont effectuées à une valeur de pH comprise entre pH = 9,0 et pH = 11,5.

**3.** Procédé selon l'une quelconque des revendications 1 ou 2, dans lequel l'étape C) est effectuée à une valeur de pH comprise entre pH = 9,5 et pH = 10,5.

**4.** Procédé selon l'une quelconque des revendications 1 à 3, dans lequel l'étape D) est effectuée à une valeur de pH comprise entre pH = 4,8 et pH = 5,6.

**5.** Procédé selon l'une quelconque des revendications 1 à 4, dans lequel l'étape E) est effectuée à une valeur de pH comprise entre pH = 3,5 et pH = 4,5.

**6.** Procédé selon l'une quelconque des revendications 1 à 5, dans lequel l'étape F) est effectuée à une valeur de pH comprise entre pH = 8,5 et pH = 10,5.

**7.** Procédé selon la revendication 1 ou la revendication 6, dans lequel la substance amphotère organique est un acide aminé avec une solubilité dans l'eau de plus de 150 g/l à 20°C.

**8.** Procédé selon la revendication 1 ou la revendication 7, dans lequel la substance amphotère organique est une ou plusieurs des substances : la glycine, l'arginine, l'alanine, la béta-alanine, la sérine, la cystéine ou la proline.

**9.** Procédé selon la revendication 1 ou la revendication 8, dans lequel la substance amphotère organique maintient la concentration de cyanate dans une solution aqueuse contenant de l'urée dans une gamme de concentration de 5 M à 8 M à des concentrations inférieures à 1,5 mM.

**10.** Procédé selon l'une quelconque des revendications 1 à 9, dans lequel dans l'étape D) une deuxième substance tampon est ajoutée au tampon aqueux, la deuxième substance tampon a au moins une valeur de pKa comprise dans les 50% centrales de la gamme définie par les valeurs de pKa d'au moins un groupe amino et d'au moins un groupe d'acide carboxylique de la substance amphotère.

**11.** Procédé selon la revendication 10, dans lequel au moins une valeur de pKa de la deuxième substance tampon est comprise entre 4,5 et 5,9.

**12.** Procédé selon la revendication 10 ou la revendication 11, dans lequel la deuxième substance tampon est un acide monocarboxylique, notamment de l'acide acétique, propanoïque, butyrique, valérique, ou de l'acide benzoïque.

**13.** Procédé selon les revendications 10 à 12, dans lequel la deuxième substance tampon est un acide dicarboxylique, notamment de l'acide succinique, adipique, pimélique, subérique ou azélaïque.

**14.** Procédé selon les revendications 10 à 12, dans lequel la deuxième substance tampon est de l'acide succinique.

**Figure 1**

pH 5.0   5.2   5.4   5.6
 P  S   P  S   P  S   P  S   mwm
— 20
10
6.8

mwm: molecular weight marker (kD)
P: precipitate
S: supernatant

**Figure 2**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0055945 A
- EP 0489780 A
- EP 0347781 A
- CA 02315750
- US 4605513 A
- US 7459425 B
- EP 2374888 A1
- US 5514646 A
- EP 0668292 A2
- WO 2009133529 A2
- US 76459425 B2
- DE 3501641

**Non-patent literature cited in the description**

- **PINES ; INOUYE.** *Mol. Biotechnol.,* 1999, vol. 12, 25-34
- **RUDOLPH ; LILIE.** *FASEB,* 1986, vol. 10, 49-56
- **DEBERNADEZ-CLARK.** *Curr. Opinion Biotech,* 2001, vol. 12, 202-207
- **WINTER et al.** *Analytical Biochemistry,* 2002, vol. 310, 148-155
- **SON et al.** *J. Microbiol. Biotechnol.,* 2008, vol. 18 (5), 983-989
- **COLE.** *J. Biol.Chem.,* 1961, vol. 236, 2670-2671
- **OIMOMI et al.** *Nephron,* 1987, vol. 46, 63-66
- *J. Biol. Chem.,* 1942, vol. 142, 705